# EUROPÄISCHE PATENTANMELDUNG

(11) **EP 1 125 583 A1**
(43) Veröffentlichungstag der Anmeldung: **22.08.2001**
(21) Anmeldenummer: 00102972.7
(22) Anmeldetag: 14.02.2000
(51) Int. Cl.: A61K 31/40, A61K 31/54, A61K 31/205, A61K 31/195, A61K 39/395

(54) **Verwendung von kompatiblen Soluten als Inhibitoren des enzymatischen Abbaus von makromolekularen Biopolymeren**

(71) Anmelder: Bitop Gesellschaft für biotechnische Optimierung MbH, D-58453 Witten (DE)
(72) Erfinder: Galinski, Erwin, Prof. Dr., 48308 Senden (DE); Kaufmann, Michael, Dr., 44388 Dortmund (DE); Schwarz, Thomas, Dr., 42799 Leichlingen (DE); Vangala, Maya-Devi, Dr., 58239 Schwerte (DE)
(74) Vertreter: Meyers, Hans-Wilhelm, Dr.Dipl.-Chem.

(57) **Zusammenfassung**

Verwendung von kompatiblen Soluten zum Schutz von Biopolymeren vor Abbau mit degradierenden Enzymen

## Beschreibung

Die vorliegende Erfindung betrifft die Verwendung von kompatiblen Soluten als Inhibitoren des enzymatischen Abbaus von makromolekularen Biopolymeren.

Unerwünschter Abbau von makromolekularen Biopolymeren wird durch die spezifische Hemmung der die Reaktion katalysierenden Enzyme verhindert.

Überraschenderweise wurde nun gefunden, dass der enzymatische Abbau von makromolekularen Biopolymeren auch durch Zugabe von kompatiblen Soluten unterdrückt werden kann, ohne dass es einer spezifischen Hemmung der den Abbau katalysierenden Enzyme bedarf.

Gegenstand der Erfindung ist daher die Verwendung kompatibler Solute als Inhibitoren des enzymatischen Abbaus von makromolekularen Biopolymeren.

### Enzymatischer Abbau biologischer Makromoleküle

Biologische Makromoleküle werden in anabolischen und katabolischen Stoffwechselprozessen auf- und abgebaut. Die enzymatische Aufspaltung organischer Makromoleküle in deren Monomerverbindungen kann durch Hydrolyse oder durch Phosphorolyse geschehen. Die Spaltung erfolgt bei der Hydrolyse unter Verbrauch von Wasser, bei der Phosphorolyse unter Verbrauch von Phosphat.

Die natürlichen Katalysatoren der Hydrolyse sind Hydrolasen. Zu den Hydrolasen zählen Lipasen, welche Fette zu Glycerin und Fettsäuren spalten, Phospholipasen, welche als Verdauungsenzyme Esterbindungen von Phosphatidylverbindungen spalten, Nukleasen, welche Nukleinsäurepolymere wie DNA und RNA spalten, Glykosidasen, welche Glykoside spalten, und Proteasen, welche die Peptidbindungen von Proteinen spalten.

Während der Proteolyse - also der Hydrolyse von Proteinen - werden die Peptidbindungen (auch Amidbindungen genannt) zwischen der α-Aminogruppe einer Aminosäure und der α-Carboxylgruppe einer zweiten Aminosäure unter Verbrauch eines Wassermoleküls gespalten. Im Falle der Spaltung eines Dipeptids - eines Peptids bestehend aus zwei Aminosäuren - werden durch Proteolyse zwei freie Aminosäuren gebildet.

Die bislang bekannten Proteasen ordnet man entsprechend ihrer Funktionsweise und ihren Substraten verschiedenen Proteaseklassen bzw. - familien zu. Folgende Proteaseklassen sind beschrieben:

**Tabelle 1:**

| Bekannte Proteaseklassen und typische Vertreter | | |
|---|---|---|
| Proteaseklasse bzw. -familie | Typische Protease | Charakteristische Aminosäuregruppe des aktiven Zentrums |
| Serin-Protease 1 | Chymotrypsin A, Trypsin, Elastase, Thrombin | Katalytische Triade Aspartat, Serin, Histidin |
| Serin-Protease 2 | Subtilisin | Katalytische Triade Aspartat, Serin, Histidin |
| Cystein-Protease | Papain, Cathepsin B | Cystein, Histidin, Aspartat |
| Aspartat-Protease | Penicillopepsin, Renin, Pepsin, Plasmin | Aspartat |
| Metallo-Protease 1 | Carboxypeptidase A, Kollagenase | Zink, Calcium, Mangan, Glutamat, Tryptophan |
| Metallo-Protease 2 | Thermolysin | Zink, Glutamat, Histidin |

Proteolysen können partiell (limitiert) oder vollständig (total) ablaufen. Bei der partiellen Proteolyse entstehen unterschiedlich große Proteinfragmente bzw. Peptide, bei der totalen Hydrolyse wird ein Protein vollständig zu Aminosäuren abgebaut. Die Proteinketten werden hier strukturspezifisch oder -unspezifisch vom Ende der Proteinstränge durch sogenannte Exoproteinasen oder nach einer Spaltung mitten im Proteinstrang durch sogenannte Endoproteinasen abgebaut.

### Technische Anwendungen der enzymkatalysierten Hydrolyse

Die enzymkatalysierte Hydrolyse wird auf vielen technischen Gebieten angewendet, wobei in der Biotechnik insbesondere die Proteolyse eine große Bedeutung besitzt. Spezifische Proteasen werden beispielsweise als biochemische Werkzeuge zur Aufklärung von Struktur-/Funktionsbeziehungen von Proteinen eingesetzt. Hierzu werden Proteine einer partiellen (limitierten) Proteolyse unterzogen und es wird geprüft, welche Eigenschaften die verbleibenden Proteinbruchstücke bzw. Peptide besitzen. Proteaseinhibitoren werden hier gezielt zum Abbruch ablaufender Proteolysen eingesetzt.

Die Proteolyse wird des weiteren zur Proteinsequenzanalyse und zur Peptidkartierung eingesetzt. Ebenfalls werden Proteasen zur Analyse der Topologie von Protein enthaltenden, biologischen Membranen und zur Solubilisierung von Membranproteinen verwendet.

Proteasen werden auch großtechnisch zur katalytischen Prozessierung von Proteinen und Peptiden eingesetzt. So werden Proteasen in Waschmitteln zur Entfernung von Proteinverunreinigungen in Textilien oder zur Reinigung technischer Oberflächen von Proteinverschmutzungen verwendet. Außerdem werden Proteasen zur industriellen Herstellung von Peptiden oder Aminosäuren aus Proteinen genutzt.

### Schutz vor enzymatischem Abbau

Biologische Makromoleküle und Polymere können vor einem enzymatischen Abbau geschützt werden.

So kann beispielsweise die Proteolyse durch die Protease hemmende, sogenannte Proteaseinhibitoren teilweise oder vollständig verhindert werden. Proteaseinhibitoren werden in zwei Klassen unterteilt:
- Niedermolekulare, spezifisch am aktiven Zentrum einer Protease bindende Hemmstoffe, welche irreversibel die Aminosäurereste im aktiven Zentrum der Proteasen derart modifizieren, dass diese ihre Funktionalität verlieren.
- Proteaseinhibitoren, welche als sogenannte Pseudosubstrate für Proteasen dienen. Proteasen werden sozusagen von ihrem eigentlichen Proteinsubstrat ablenkt und der Reaktionslösung stöchiometrisch entzogen. Die Proteasen werden hier zwar nicht zerstört, aber spezifisch entfernt. Klasse 2 Proteaseinhibitoren sind ebenfalls solche, welche den Enzymen für ihre Aktivität essentielle Cofaktoren entziehen.

Zur erstgenannten Gruppe zählen z.B. die Serin-Protease-Inhibitoren Diisopropyl Phosphofluoridate (DFP) und Phenylmethansulfonyl Fluoride (PMSF). Aspartat Proteasen werden durch Diazoacetyl Verbindungen und durch Pepstatin inaktiviert. Metallo-Proteasen werden generell durch metall-chelatisierende Reagenzien inhibiert. Carboxypeptidase A und B werden spezifisch durch aus Kartoffeln isolierbare Hemmstoffe und Thermolysin durch Phosphoramidon gehemmt.

Zur zweiten Gruppe der Proteaseinhibitoren zählen z.B. Pankreas Trypsin Inhibitor, Sojabohne Trypsin Inhibitor, α-Proteaseinhibitor und der universelle Proteaseinhibitor α-2-Makroglobulin.

G. Salvesen und H. Nagase (1989) geben einen Überblick über die nach dem Stand der Technik genutzten Klasse 1 und Klasse 2 Inhibitoren [Inhibition of proteolytic enzymes in Proteolytic enzymes: A practical approach (Herausgeber; R.J. Beynon und J.S. Bond, IRL Press Oxford)].

Klasse 1 Proteaseinhibitoren haben aufgrund ihres Wirkprinzips alle den Nachteil, dass sie grundsätzlich mit allen Proteinen und nicht nur mit Proteasen bzw. deren aktiven Zentren reagieren können. Klasse 1 Proteaseinhibitoren besitzen somit für biologische Systeme und Organismen ein toxisches Potential. Klasse 2 Proteaseinhibitoren weisen u.a. den Nachteil auf, dass sie die Proteasen nur stöchiometrisch und reversibel hemmen. Eine Proteolyse bleibt prinzipiell immer möglich.

Den bis hierher beschriebenen Verfahren zum Schutz vor enzymatischem Abbau ist gemein, dass der jeweils dafür verantwortliche Biokatalysator spezifisch gehemmt wird.

Die Proteolyse von Proteinen kann auch durch Proteindenaturierung verhindert werden. Hierbei wird die Sekundär-, Tertiär- sowie Quartär-Struktur aller Proteine vollständig zerstört, so dass spezifische Proteasen keine Aktivität mehr aufweisen. Derartige Denaturierungen werden durch chaotrope Reagenzien, wie z.B. Harnstoff oder Guanidiniumhydrochlorid, bzw. durch Detergenzien, wie Natriumdodecylsulfat, erreicht. Besonders nachteilig bei diesen Verfahren ist, dass eine Proteolyse zwar verhindert wird, aber die zu schützenden Proteine in den meisten Fällen ihre Funktion einbüssen.

Keine der hier aufgeführten Methoden zielt auf die Stabilisierung der jeweiligen Substrate ab.

### Beschreibung der Erfindung

Überraschenderweise hat sich gezeigt, dass die Verwendung von kompatiblen Soluten den Abbau von makromolekularen Biopolymeren, insbesondere von Makromolekülen, Proteinen, Lipiden oder Nucleinsäuren, mit degradierenden Enzymen verhindert.

Vorzugsweise werden erfindungsgemäß als kompatible Solute Substanzen gewählt aus der Gruppe bestehend aus Ectoin, Derivaten des Ectoins wie Hydroxyectoin, Prolin, Betain, Glutamin, cyclisches Diphosphoglycerat, Mannosylglycerat, Derivaten des Mannosylglycerats wie Mannosylglyceramid, Di-myo-Inositolphosphat, Diglycerolphosphat, N_{γ}-Acetylornithin, Trimethylamine-N-oxid oder Kombinationen davon eingesetzt werden.

Die Verwendung der kompatiblen Solute in einer Konzentration von 0,05 bis 2,0 M ist bevorzugt. Weiter bevorzugt ist bei Verwendung von Ectoin eine Konzentration von 0,1 - 1 M, insbesondere 0,1 - 0,5 M. Bei Verwendung anderer Solute sind 0,4 - 1,5 M, insbesondere 0,4 - 1,2 M bevorzugt.

Erfindungsgemäß wird ein Verfahren zum Schutz von Biopolymeren vor dem Abbau durch degradierende Enzyme bereitgestellt, bei dem zu einer die Biopolymere enthaltenden Probe kompatible Solute zugefügt werden.

Gegebenenfalls erfolgt nach Zugabe des kompatiblen Solutes oder der kompatiblen Solute eine Inkubation, woraufhin sich gegebenenfalls Weiterverarbeitungsschritte, wie Zellaufschluß und Isolierung des abbaubaren Biopolymers anschließen.

Insbesondere ist die Probe ein biotechnischer Ansatz zur Herstellung von Proteinen oder Nucleinsäuren.

Die der Erfindung zugrunde liegenden Untersuchungen haben nun überraschend gezeigt, dass durch Einsatz kompatibler Solute der enzymatische Abbau verhindert werden kann, wobei erstaunlicherweise weder die Funktionalität des zu schützenden Biopolymers noch die des abbauenden Enzymes verloren gehen. Die Resultate zeigen außerdem, dass Makromoleküle vor einem enzymatischen Angriff geschützt werden können, während kleine Proteine und Peptide nicht geschützt werden.

Der Abbau scheint also bei Einwirkung kompatibler Solute nicht durch eine spezifische Inhibition oder Eliminierung der degradierenden Enzyme, sondern vielmehr über eine möglicherweise unspezifische Stabilisierung der makromolekularen Substrate selbst verhindert zu werden. Durch die Zugabe von kompatiblen Soluten wird das Biopolymer scheinbar in seiner Struktur so verändert, dass das abbauende Enzym das Biopolymer nicht mehr "erkennt". Enzymatischer Abbau von Biomolekülen wird somit wahrscheinlich durch sterische Enzym-Substrat-Inkompatibilität unspezifisch unterdrückt.

Die Anwendung der Erfindung hat insbesondere für Anwendungen bei Proteinen den immensen Vorteil, dass eine einzige Inhibitionslösung universell einsetzbar wird. Häufig enthalten Proteinlösungen ein größeres Spektrum an unerwünschten Proteasen, was nach dem Stand der Technik für jeden Proteasetyp einen spezifischen Inhibitor erfordert und die Verwendung von Inhibitionsmixturen nach sich zieht. Dieses Vorgehen und die damit verbundenen Nachteile können durch das erfindungsgemäße Verfahren umgangen werden.

Im Zusammenhang mit den hier beschriebenen Wirkungen stehen auch Wirkungen der kompatible Solute als Arzneimittel. Erfindungsgemäß können die kompatiblen Solute zur Herstellung von Arzneimitteln zur Behandlung von Erkrankungen eingesetzt werden, die durch enzymatischen Abbau von Biopolymeren und von Bioploymeren gebildeten Strukturen, wie Zellen, Organellen oder Gewebe, verursacht werden und im kausalen Zusammenhang mit pathologischen Erscheinungen stehen. Dazu gehören insbesondere Erkrankungen, wie Erkrankungen des Immunsystems, wie Autoimmunerkrankungen, Insulin abhängige Diabetes melitus, Graves-Krankheit, Hashimotos-Krankheit, nachteilige Nebenwirkungen von Strahlungsbehandlungen, Entzündungsprozesse, Transplantabstoßung, HIV-Infektionen bzw. retrovirale Infektionen (z.B. Herpes), Gewebeverletzung/Wundheilung, akute und chronische Entzündung, akute Pankreatitis, Schockzustände, fibrinolytische Blutung, Herzkrankheiten (z.B. Infarkt), Alzheimer's Krankheit, neuronale Degeneration, Krankheiten von Leber, Skelett und Muskel, Bluthochdruck, Metastasenbildung von Krebszellen und Hautkrebs.

### Beispiele

Die Wirksamkeit des der Erfindung zugrunde liegenden Prinzips wurde durch Hemmung der limitierten Proteolyse von Antikörpern dokumentiert. Die Experimente wurden mit folgenden Antikörpern oder Konjugaten ausgeführt: Humanes lgG: Sigma Product No. 14506 Lot 037H8816; Rinder IgG; Serva; Cohn-Fraktion II Product No. 22550; Monoklonaler Maus Anti-Human lgG: DAKO Klon A57H Code No. Mo828 Lot 076 lgM kappa: Kaninchen Anti-Maus lgG + lgM (H+L): Dianova Code No. 315-035-058 Lot 39605.

Es wurden limitierte Proteolysen des humanen lgG bei einer Antikörperkonzentration von 0,5 mg/ml und einer Pepsinkonzentration von 5 µg/ml bei 37°C in 100 mM Natriumacetat pH 3 durchgeführt. Die Inkubationen erfolgten ohne Zusätze unter 0,5 M Ectoin, unter 0,5 M Hydroxyectoin sowie unter einer Mischung aus 0,25 M Ectoin und 0,25 M Hydroxyectoin. Der Verlauf der limitierten Proteolysen wurde im Anschluss an die Inkubationen in 12%igen SDS-Gelen unter reduzierenden Bedingungen quantifiziert.

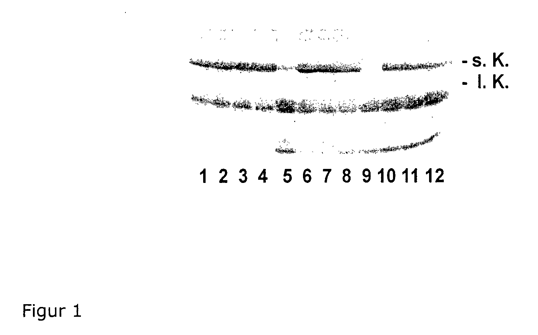

Aus Figur 1 ergibt sich, dass die Hydrolyse der schweren Kette sowohl durch Ectoin als auch durch Hydroxyectoin signifikant gehemmt wird.

Figur 1 zeigt die limitierte Proteolyse der schweren Ketten (s. K.) eines humanen Antikörpers (lgG) durch Pepsin. Bahnen 1, 5, 9: mit Wasser: Bahnen 2, 6, 10: mit Ectoin; Bahnen 3, 7, 11: mit Ectoin und Hydroxyectoin; Bahnen 4, 8, 12: mit Hydroxyectoin; Bahnen 1, 2, 3, 4: ohne Inkubation; Bahnen 5, 6, 7, 8; nach 15 min. Inkubation; Bahnen 9, 10, 11, 12; nach 180 min. Inkubation.

Die Hemmung der Proteolyse von Antikörpern durch Ectoin wurde zusätzlich mittels Enzyme Linked Immunosorbant Assay (ELISA) gezeigt. Dazu wurden 96-Well-ELISA-Platten über Nacht mit 0,5 µg humanem lgG in 100 µl 0,9% NaCl bei 4°C beschichtet. Im Anschluß daran wurden die Platten 3 x in 50 mM Tris, 0,8% NaCl, 0,02% KCI, pH 74 C(T-TBS) gewaschen und mit 200 µl 1% Gelatine in TBS für 2 Stunden bei 37°C blockiert. Nach dreimaligem Waschen in T-TBS erfolgte eine Inkubation mit einem monoklonalen Maus-anti-Human-lgG 1 : 250 in TBS verdünnt mit 50 µl pro Well für eine Stunde bei 37°C. Nach viermaligem Waschen mit T-TBS erfolgte eine Inkubation mit einem Peroxidase konjugierten Kaninchen anti-Maus-Antikörper 1 : 5000 in TBS verdünnt mit 100 µl pro Well für eine Stunde bei 37°C. Nach weiterem viermaligem Waschen erfolgte die Enzymreaktion durch Zugabe von 100 µl pro Well Substrat. Als Substrat diente 4,8 mg Phenylendiamin in 3 ml 0,2 M Na₂HPO₄, 3 ml 0,1 M Citronensäure, 6 ml Wasser und 5 µl H₂0₂, Die Reaktion wurde nach ca. 10 Minuten durch Ansäuern mit 100 µl pro Well 0,07 M Schwefelsäure gestoppt und die Platten in einem ELISA-Autoreader quantifiziert. Alle drei Proteinkomponenten wurden vor dem Experiment für zwei Stunden bei 37°C mit bzw. ohne Zugabe von 0.5 M Ectoin durch Pepsin proteolysiert. Wie aus Tabelle 2 ersichtlich ist, konnten zwei Antikörper gegen den proteolytischen Verdau durch Zugabe von 0,5 M Ectoin wirkungsvoll geschützt werden. Das Antikörperkonjugat wurde nicht durch Pepsin angegriffen. Die Behandlung wurde in analoger Weise mit Hydroxyectoin, Prolin und Betain durchgeführt.

**Tabelle 2:**

| Restsignal im ELISA nach angegebener Vorbehandlung der einzelnen Antikörper | | | |
|---|---|---|---|
| Vorbehandelter Antikörper | | | |
| | humaner lgG | monoklonaler Maus-Antikörper | Peroxidase Konjugat |
| ohne Behandlung | 100% | 100% | 100% |
| mit Ectoin | 85% | 97% | 98% |
| mit Pepsin | 6% | 59% | 104% |
| mit Ectoin und Pepsin | 91% | 98% | 121% |

Die Konzentrationsabhängigkeit des Effektes vom kompatiblen Solut wurde am Beispiel von Ectoin, Hydroxyectoin, Prolin und Betain bestimmt. Hierbei wurde das humane Immunglobulin wie beschrieben proteolysiert und der Effekt im ELISA quantifiziert.

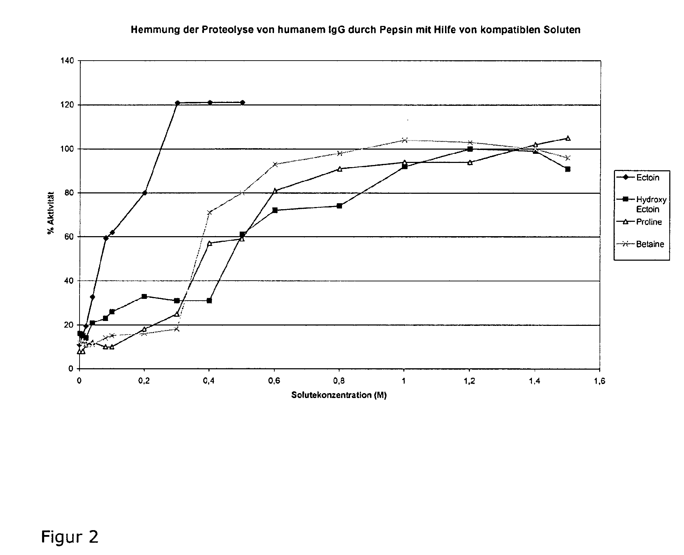

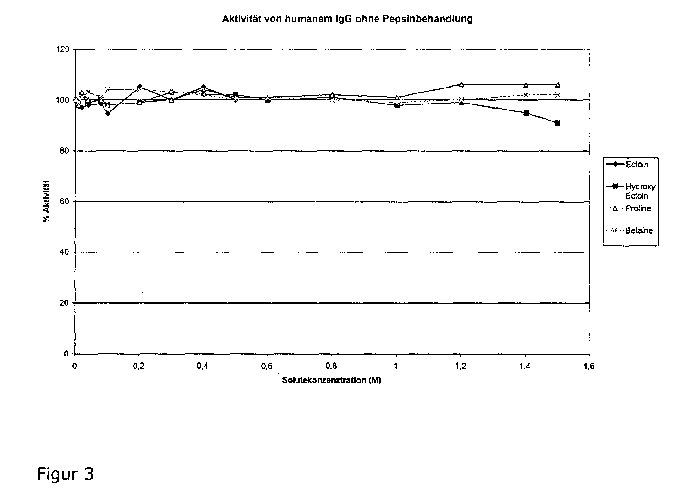

Figur 2 zeigt die Konzentrationsabhängigkeit der Stabilisierung eines humanen Antikörpers gegen Proteolyse durch Pepsin in Gegenwart steigender Konzentration an Ectoin, Hydroxyectoin, Prolin, Betain. Die Figur 3 zeigt das Ergebnis der Kontrollversuche ohne Zugabe von Pepsin zum Gemisch aus Antikörper und kompatiblem Solut.

Die Experimente lassen prinzipiell zwei Erklärungsmöglichkeiten zu. Zum einen könnten die kompatiblen Solute die Protease inhibieren. Zum anderen könnten die Solute erfindungsgemäß über die Stabilisierung der nativen, kompakteren Konformation des Antikörpermoleküls den Angriff der Protease aus sterischen Gründen verhindern. Es wurde daher der Effekt von Ectoin und Hydroxyectoin auf die Aktivität von Pepsin anhand der Hydrolyse eines synthetischen, niedermolekularen Hexapeptids spektrophotometrisch bestimmt [Schinaith, E.: Clin. Biochem. 22, 91-98 (1989)]. Die enzymkinetischen Messungen mit Leu-Ser-p-NitroPhe-Nle-Ala-Leu-Methylester als Substrat zeigten, dass weder Ectoin noch Hydroxyectoin die Protease inhibieren. Dies ist ein deutliches Indiz dafür, dass die Hemmung der Antikörperproteolysen erfindungsgemäß auf sterische Effekte zurückzuführen ist.

## Patentansprüche

1. Verwendung von kompatiblen Soluten zum Schutz von Biopolymeren vor Abbau durch degradierende Enzyme.

2. Verwendung nach Anspruch 1, wobei die Biopolymere Makromoleküle, Lipide, Proteine, Fette oder Nucleinsäuren sind.

3. Verwendung nach Anspruch 1 und/oder 2, wobei die degradierenden Enzyme Hydrolasen, insbesondere Proteasen, oder Phosphorylasen sind.

4. Verwendung nach einem der Ansprüche 1 bis 3, wobei als kompatible Solute Substanzen gewählt aus der Gruppe bestehend aus Ectoin, Derivaten des Ectoins wie Hydroxyectoin, Prolin, Betain, Glutamin, cyclisches Diphosphoglycerat, Mannosylglycerat, Mannosylglyceramid, Di-myo-inositol-1,1'-Phopshat, Diglycerolphosphat, N_{γ} -Acetylornithin, Trimethylamine-N-oxid oder Kombinationen davon eingesetzt werden.

5. Verwendung nach mindestens einem der Ansprüche 1 bis 4, wobei die kompatiblen Solute in einer Konzentration von 0,05 bis 2 M eingesetzt werden.

6. Verfahren zum Schutz von Biopolymeren vor Abbau durch degradierende Enzyme, wobei zu einer die Biopolymere enthaltenden Probe kompatible Solute zugefügt werden.

7. Verfahren nach Anspruch 6, wobei nach Zugabe des kompatiblen Soluts oder der kompatiblen Solute eine Inkubation erfolgt, woraufhin sich gegebenenfalls Weiterverarbeitungsschritte, wie Zellaufschluß und Isolierung des abbaubaren Biopolymers anschließen.

8. Verfahren nach Anspruch 6 und/oder 7, wobei die Probe ein biotechnologischer Ansatz zur Herstellung von Proteinen oder Nucleinsäuren ist.

9. Verwendung von kompatiblen Soluten zur Herstellung eines Arzneimittels zur Behandlung von Erkrankungen, die durch den Abbau von Biopolymeren und von Bioploymeren gebildeten Strukturen, wie Zellen, Organellen oder Gewebe, durch degradierende Enzyme verursacht werden.

10. Verwendung nach Anspruch 9, wobei die Erkrankungen ausgewählt sind aus der Gruppe bestehend aus Erkrankungen des Immunsystems, wie Autoimmunerkrankungen, Insulin abhängige Diabetes melitus, Graves-Krankheit, Hashimotos-Krankheit, nachteilige Nebenwirkungen von Strahlungsbehandlungen, Entzündungsprozesse, Transplantabstoßung, HIV-Infektionen bzw. retrovirale Infektionen (z.B. Herpes), Gewebeverletzung/Wundheilung, akute und chronische Entzündung, akute Pankreatitis, Schockzustände, fibrinolytische Blutung, Herzkrankheiten (z.B. Infarkt), Alzheimer's Krankheit, neuronale Degeneration, Krankheiten von Leber, Skelett und Muskel, Bluthochdruck, Metastasenbildung von Krebszellen und Hautkrebs.
